(19) Europäisches Patentamt
European Patent Office
Office européen des brevets

(11) EP 1 637 125 A2

(12) **DEMANDE DE BREVET EUROPEEN**

(43) Date de publication:
**22.03.2006 Bulletin 2006/12**

(51) Int Cl.:
***A61K 8/891*** *(2006.01)* ***A61Q 1/06*** *(2006.01)*

(21) Numéro de dépôt: **05291219.3**

(22) Date de dépôt: **07.06.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **24.08.2004 FR 0451896**
**21.10.2004 FR 0411209**

(71) Demandeur: **L'OREAL**
**75008 Paris (FR)**

(72) Inventeurs:
- **Bavouzet, Bruno**
**94250 Gentilly (FR)**
- **Le Chaux, Laure**
**94240 L'Hay les Roses (FR)**

(74) Mandataire: **Chantraine, Sylvie Hélène**
**L'OREAL - D.I.P.I.**
**25-29, Quai Aulagnier**
**92600 Asnieres (FR)**

(54) **Produit de maquillage bicouche de tenue améliorée, ses utilisations et kit de maquillage contenant ce produit**

(57) L'invention se rapporte à un produit cosmétique de maquillage contenant une première et une seconde compositions, la première composition comprenant un milieu physiologiquement acceptable, et la seconde composition comprenant au moins un polymère siliconé de viscosité mesurée selon la norme ASTM D-445 comprise entre 5 et 10 000 cSt.

L'invention se rapporte également à procédé de maquillage ainsi qu'à un kit de maquillage contenant ledit produit.

Le produit de maquillage est en particulier un rouge à lèvres.

EP 1 637 125 A2

**Description**

La présente invention se rapporte à une composition cosmétique de soin ou de maquillage de la peau présentant des propriétés de tenue améliorées tout en ayant des propriétés de non migration, de non filant et de non collant satisfaisantes.

Le produit cosmétique comprend au moins deux compositions qui peuvent être appliquées successivement sur la peau, sur les paupières, sur les lèvres et sur les phanères comme les ongles, les sourcils, les cils ou les cheveux. En particulier, le produit cosmétique peut être un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un vernis à ongles ou encore un produit de maquillage du corps ou de coloration de la peau.

Les compositions sans transfert connues (US 6 074 654, WO 02/067877) sont généralement à base de résines de silicone et d'huiles de silicone volatiles et, bien que présentant des propriétés de tenue améliorées, ont l'inconvénient de laisser sur la peau et les lèvres, après évaporation des huiles de silicone volatiles, un film qui devient inconfortable au cours du temps (sensation de dessèchement et de tiraillement), écartant un certain nombre de femmes de ce type de rouge à lèvres.

Il a été proposé d'appliquer sur une première composition contenant une dispersion de particules de polymère filmogène acrylique, une deuxième composition comprenant un mélange d'une huile siliconée phénylée de viscosité égale à 20 cSt et une huile siliconée phénylée de viscosité égale à 10 000 cSt (voir la demande de brevet EP 1 249 223). Cette composition présente cependant le désavantage de migrer et de devenir collante. En outre, le produit cosmétique décrit dans ce document ne présent pas une tenue satisfaisante.

Dans la demande de brevet WO-A-97/17057 la société Procter et Gamble décrit une méthode pour augmenter les propriétés de tenue et de non transfert consistant à appliquer deux compositions l'une sur l'autre. Ces deux compositions satisfont aux critères physico-chimiques suivants :

- la composition appliquée en premier est dotée d'un paramètre de solubilité global d'Hildebrand inférieur à 8,5 $(cal/cm^3)^{1/2}$, et
- la deuxième composition contient des huiles dont le coefficient de partage calculé ClogP est au moins égal à 13.

Cependant, les compositions données en exemple procurent un maquillage inconfortable.

Dans la demande WO 02/067877, il est décrit une méthode pour améliorer les propriétés esthétiques d'une composition non transfert consistant à appliquer sur un film de composition non transfert, une deuxième composition qui ne détruit pas le film de la première. Les produits décrits dans ce document ont une odeur désagréable et sont collants.

Enfin, le brevet US-A-6 001 374 de Nichols propose un maquillage des lèvres consistant à appliquer une composition de base contenant une résine soluble dans l'alcool et insoluble dans l'eau, sur laquelle on dépose une composition contenant un composé siliconé. Les composés siliconés cités en exemple, notamment la polydiméthylsiloxane de viscosité 20 000 000 cSt et de poids moléculaire 500 000, en solution dans une polydiméthylsiloxane de viscosité 5 cSt et de poids moléculaire 800, dans les proportions massiques 15/85 (produit commercialisé sous la référence SF 1236), sont trop collants.

La présente invention a pour but de proposer un produit cosmétique, en particulier un produit de maquillage, comprenant deux compositions à appliquer l'une sur l'autre, le produit étant tel que la tenue est améliorée, les propriétés de non migration et de non collant sont satisfaisantes. La présente invention fournit également un produit cosmétique de tenue satisfaisante dont les propriétés de non collant sont améliorées.

Selon un de ses aspects, la présente invention a pour objet un produit cosmétique, notamment un produit de maquillage comprenant une première et une seconde compositions destinée à être appliquée sur la première, la première composition comprenant un milieu physiologiquement acceptable, la seconde composition comprenant au moins un premier polymère siliconé de viscosité mesurée selon la norme ASTM D-445 comprise entre 5 et 10 000 cSt, voir entre 10 et 10 000 cSt. Dans la présente demande, l'expression « compris entre » inclus les bornes de la plage.

La première composition peut contenir un polymère acrylique. Elle peut être solide. Par composition solide, on entend notamment un stick dans le cas d'un rouge à lèvres ou d'un fond de teint.

Par produit de maquillage, on entend un produit contenant un agent colorant permettant le dépôt d'une couleur sur une matière kératinique (la peau, les lèvres ou les phanères) de personne humaine par l'application sur la matière kératinique de produits tels que des rouges à lèvres, des fards, des eye-liners, des fonds de teint ou des autobronzants, des produits de maquillage semi permanent (tatouage).

Par polymère siliconé, on entend un polymère obtenu à partir d'au moins un monomère comprenant au moins un atome de silicium.

Un dépôt du produit selon l'invention résultant de l'application successive des première et deuxième compositions, présente avantageusement une tenue supérieure ou égale à 60%, par exemple 65%, voir même 70%.

La tenue est mesurée selon la méthode suivante.

On prépare trois lames de verres recouvertes d'une feuille de collagène comme suit.
A une température de 28°C, on prépare une feuille de collagène (Nippi casing Grade) de 4,6 cm par 8,5 cm de côté et on la met à conditionner 3 heures à 90% d'Humidité Relative (HR). On remet la feuille de collagène à l'air libre et on la fixe immédiatement fermement et entièrement sur une lame de verre 4,6 cm par 7,6 cm. On attache la feuille de collagène sur l'envers de la lame avec du scotch 3M. La surface du collagène doit être plane et exempte de pli. Chaque lame est laissée en conditions ambiantes pendant 24 heures, avant de réaliser le test.
On coupe un rectangle de 4,6 cm par 7,6 cm dans une assiette de Styrofoam (type Amoco Selectables Plastics DL Tableware) au moyen d'un couteau et d'une règle en suivant le contour d'une lame de verre. On applique le produit selon l'invention sur chaque lame de verre et sur le rectangle de Styrofoam comme suit. On applique la première composition à l'aide d'un applicateur mécanique de 25μm (bar coater). On laisse sécher 30 minutes, puis on applique la deuxième composition sur le dépôt de la première.
On applique le produit sur les lames de verre recouvertes de collagène en appliquant la première composition à l'aide d'un applicateur mécanique de 25μm (bar coater). On laisse sécher 30 minutes, puis on applique la deuxième composition sur le dépôt de la première. On laisse les plaques en conditions ambiantes pendant 30 minutes.
On applique 3 gouttes d'huile (environ 0,075 g) étalées avec un pinceau sur chacune des trois lames recouvertes de collagène. On sèche l'excédent avec un papier Kimwipes et on laisse les lames 30 minutes à température ambiante. On coupe 3 disques de Styrofoam blanc de 3,8 cm de diamètre.
On attache fermement avec du double face le disque de Styrofoam blanc au bout d'une masse de 2kg et, en exerçant une pression de 175g/cm$^2$, on pose doucement le poids à la surface d'une plaque (côté produit) et on procède, en 3 à 5 secondes, à une rotation d'un tour et demi du poids sur lui-même et ce, en maintenant la pression initale. On remonte le poids et on récupère le disque de Styrofoam. La mesure est effectuée pour chaque lame de verre avec un disque de Styrofoam propre.
On mesure ensuite le pourcentage de réflectance :

- o du dépôt de produit appliqué sur l'échantillon rectangulaire de Styrofoam (référencé A),
- o du disque propre de Styrofoam blanc (référencé B)
- o du disque décollé du poids après avoir appliqué la pression sur la lame enduite de produit cosmétique (référencé C)

La réflectance est mesurée sur une gamme de longueur d'onde comprise entre 400 et 700nm à l'aide d'un analyseur spectral (ouverture 25 mm de diamètre) avec un illuminant D65/10degrés. On choisit la longueur d'onde du minimum de réflectance pour le disque 'taché'. A cette longueur d'onde, on calcule la tenue selon l'équation

$$100 * (1 - [(C-B)/(A-B)])$$

La tenue est égale à la moyenne des trois mesures.
Selon un de ses aspects, la présente invention a pour objet un produit cosmétique, notamment de maquillage, comprenant une première composition et une seconde composition destinée à être appliquée sur la première, la première composition comprenant un milieu physiologiquement acceptable, et la seconde composition comprenant au moins un premier polymère siliconé de viscosité mesurée selon la norme ASTM D-445 comprise entre 5 et 10 000 cSt, le premier polymère siliconé étant tel que lorsqu'il est en quantité suffisante dans la seconde composition, le rapport entre i) la tenue d'un dépôt dudit produit résultant de l'application successive des première et seconde compositions et ii) la tenue d'un dépôt de la première composition est supérieur à au moins 1,1, la tenue d'un dépôt de ladite première composition étant supérieure ou égale à 30%.
La première composition peut contenir un polymère acrylique. Elle peut être solide.
Le rapport entre i) la tenue d'un dépôt dudit produit résultant de l'application successive des première et seconde compositions et ii) la tenue d'un dépôt de la première composition peut être supérieur à au moins 1,12, par exemple 1,15, voire 1,17, voire même 1,20.
Selon cet aspect de l'invention, mais aussi selon les autres aspects présentés ci-après, la tenue de la première composition peut être supérieure ou égale à 40%, voire 50%, voire même 55%, voire même encore 60%.
La tenue est mesurée selon la méthode décrite précédemment.
Selon un deuxième aspect, la présente invention a pour objet un produit cosmétique, notamment de maquillage, comprenant une première composition et une seconde composition destinée à être appliquée sur la première, la première composition comprenant un milieu physiologiquement acceptable, et la seconde composition comprenant au moins un premier polymère siliconé choisi parmi les polydiméthylsiloxanes de viscosité mesurée selon la norme ASTM D-445 comprise entre 5 et 10 000 cSt, voir entre 10 et 10 000 cSt, et au moins un deuxième polymère siliconé choisi parmi les polydiméthylsiloxanes de viscosité mesurée selon la norme ASTM D-445 comprise entre 5 000 et 300 000 cSt et

supérieure à la viscosité dudit premier polymère.

La première composition peut contenir un polymère acrylique. Elle peut être solide.

Selon encore un autre aspect, la présente invention concerne un produit cosmétique, notamment de maquillage, comprenant une première composition et une seconde composition destinée à être appliquée sur la première, la première composition comprenant un milieu physiologiquement acceptable, et la seconde composition contenant un premier polymère siliconé de viscosité mesurée selon la norme ASTM D-445 comprise entre 50 et 1 000 cSt et un second polymère siliconé de viscosité mesurée selon la norme ASTM D-445 comprise entre 30 000 et 300 000 cSt.

La première composition peut contenir un polymère acrylique. Elle peut être solide.

Selon un dernier aspect, la présente invention a pour objet un produit cosmétique, notamment de maquillage, comprenant une première composition et une seconde composition destinée à être appliquée sur la première, la première composition comprenant un milieu physiologiquement acceptable, et la seconde composition comprenant au moins un premier polymère siliconé de viscosité mesurée selon la norme ASTM D-445 comprise entre 10 et 10 000 cSt, le premier polymère siliconé étant tel que lorsqu'il est en quantité suffisante dans la seconde composition, la tenue d'un dépôt dudit produit résultant de l'application successive des première et seconde compositions, est supérieure à 65%.

La première composition peut contenir un polymère acrylique. Elle peut être solide.

La présente invention a également pour objet un procédé de maquillage de la peau et/ou des lèvres et/ou des phanères consistant à appliquer sur la peau, les lèvres et/ou les phanères une première couche d'une première composition comprenant un milieu physiologiquement acceptable, puis à appliquer sur tout ou partie de ladite première couche, une seconde couche d'une seconde composition comprenant au moins un polymère siliconé de viscosité mesurée selon la norme ASTM D-445 comprise entre 5 et 10 000 cSt, voir entre 10 et 10 000 cSt, et la tenue d'un dépôt résultant de l'application successive de la première composition et de la deuxième composition étant supérieure à 65%.

La première composition peut contenir un polymère acrylique. Elle peut être solide.

La présente invention concerne également l'utilisation d'une deuxième composition comprenant au moins un polymère siliconé de viscosité mesurée selon la norme ASTM D-445 comprise entre 5 et 10 000 cSt, voir entre 10 et 10 000 cSt, destinée à être appliquée sur une première composition comprenant un milieu physiologiquement acceptable, pour obtenir un produit cosmétique dont un dépôt résultant de l'application successive de la première et de la deuxième composition présente une tenue supérieure à 65%.

La première composition peut contenir un polymère acrylique. Elle peut être solide.

## Deuxième composition

La deuxième composition comprend au moins un premier polymère siliconé de viscosité mesurée selon la norme ASTM D-445 comprise entre 10 et 10 000 cSt.

La deuxième composition a avantageusement une viscosité à 25 °C, mesurée avec un viscosimètre rotatif Brookfield RV, comprise entre 3 et 25 Pa.s.

La viscosité dynamique de la composition est mesurée avec un viscosimètre Brookfield RV, qui peut être équipé de différents mobiles en fonction de l'ordre de grandeur de la viscosité que l'on veut mesurer. La composition est versée dans un bécher de 600 ml, qui est thermostaté pendant 2 heures à 25°C. La mesure, donnée en centipoises est effectuée après 10 minutes de cisaillement. La vitesse de rotation du mobile est de 200 s-1.

On mesure également la densité de la composition à l'aide d'un pycomètre à 25°C, pour convertir en Pa.s la viscosité mesurée initialement en centipoises.

Selon un premier mode de réalisation, la deuxième composition contient un seul polymère siliconé de viscosité comprise entre 10 et 10 000 cSt, de préférence entre 50 et 5 000 cSt, de préférence entre 100 et 1 000 cSt et sa viscosité à 25 °C, mesurée avec un viscosimètre rotatif Brookfield RV, est comprise entre 3 et 6 Pa.s.

Le demandeur a en effet découvert qu'en souhaitant préparer une deuxième composition de viscosité comprise entre 3 et 6 Pa.s, le choix d'un seul polymère tel que sa viscosité est comprise 10 et 10 000 cSt est nécessaire pour augmenter la tenue et diminuer le collant du produit cosmétique selon l'invention.

Selon un deuxième mode de réalisation, la seconde composition comprend au moins deux polymères siliconés de viscosités différentes comprises entre 10 et 300 000 cSt.

Notamment, la seconde composition comprend au moins un premier polymère siliconé de viscosité comprise entre 10 et 10 000 cSt, et au moins une deuxième polymère siliconé de viscosité supérieure à celle du premier polymère siliconé et comprise entre 5 000 et 300 000 cSt.

Le deuxième polymère siliconé a une masse molaire en poids inférieure à 200 000 g/mol. La masse moléculaire du composé siliconé est mesurée par chromatographie par perméation de gel (GPC).

Dans ce mode réalisation, la viscosité à 25°C de la seconde composition est de préférence comprise entre 5 et 25 Pa.s, de préférence encore entre 8 et 15 Pa.s. En effet, le demandeur a découvert que, pour obtenir une composition dont la viscosité est comprise dans cette gamme, le choix de deux polymères de viscosités particulières permet d'améliorer la tenue et de diminuer le collant du produit cosmétique selon l'invention.

Selon un mode de réalisation, la deuxième composition est transparente.

On entend par « composition transparente », une composition transparente à translucide, c'est-à-dire telle qu'elle transmet au moins 40 % de lumière à une longueur d'onde de 750 nm, et de préférence au moins 50 % de lumière. La mesure de la transmission est effectuée avec un spectrophotomètre UV-visible Cary 300 Scan de la société Varian selon le protocole suivant :

On coule la composition au-dessus de sa température de fusion dans une cuve à spectrophotomètre à section carrée dont le côté a une longueur de 10 mm.
L'échantillon de la composition est ensuite refroidi pendant 24 heure à 35 °C puis maintenu dans une enceinte thermostatée à 20 °C pendant 24 heures.
On mesure ensuite la lumière transmise à travers l'échantillon de la composition au spectrophotomètre par balayage de longueurs d'onde allant de 700 nm à 800 nm, la mesure étant faite en mode transmission.
On détermine alors le pourcentage de lumière transmise à travers l'échantillon de la composition à la longueur d'onde de 750 nm.

Le premier polymère siliconé de viscosité comprise entre 10 et 10 000 cSt peut répondre à la formule (A):

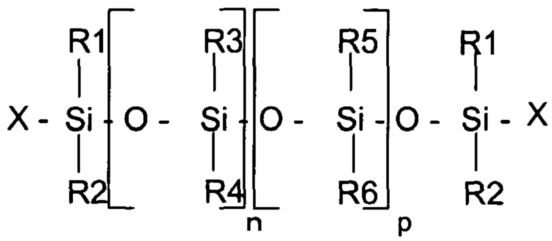

(A)

dans laquelle :

$R_1$, $R_2$, $R_5$ et $R_6$ sont, ensemble ou séparément, un radical alkyle ayant 1 à 6 atomes de carbone, éventuellement substitué par au moins un atome de fluor,
$R_3$ et $R_4$ sont, ensemble ou séparément, un radical alkyle ayant de 1 à 6 atomes de carbone ou un radical aryle,
X est un radical alkyle ayant de 1 à 6 atomes de carbone, un radical hydroxyle, un radical vinyle, allyle ou un radical alkoxyle ayant de 1 à 6 atomes de carbone,
n et p étant choisis de manière à ce que le polymère siliconé a une viscosité à 25°C, mesurée selon la norme ASTM D-445 comprise entre 10 et 10 000 cSt.
p est de préférence égal à 0.

Le premier polymère siliconé de viscosité comprise entre 10 et 10 000 cSt peut être choisi parmi les polyalkylsiloxanes, les polyarylsiloxanes, les polyalkylarylsiloxanes et leurs mélanges.

Le premier polymère siliconé de viscosité comprise entre 10 et 10 000 cSt peut être notamment parmi :

- les polydiméthylsiloxanes (PDMS);
- les polydiméthylsiloxanes comportant des groupements alkyle, alcoxy ou phényle, pendant ou en bout de chaîne siliconée, groupements ayant de 2 à 24 atomes de carbone ;
- les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les polyalkylsiloxanes selon l'invention incluent les polydiméthylsiloxanes, les copolymères (polydiméthylsiloxane)(méthylvinylsiloxane), les poly(diméthylsiloxane)(diphényl)siloxanes, les copolymères poly(diméthylsiloxane)(diphényl)(méthylvinylsiloxane), et leurs mélanges

Le premier polymère siliconé de viscosité comprise entre 10 et 10 000 cSt est avantageusement une polydiméthylsiloxane.

Le premier polymère siliconé de viscosité comprise entre 10 et 10 000 cSt a de préférence une viscosité mesurée selon la norme ASTM D-445, comprise entre 50 et 5 000 cSt, de préférence entre 100 et 3 000 cSt, notamment

entre 100 et 1 000 cSt, par exemple égale à 350 cSt.

Selon un des modes de réalisations de l'invention, la seconde composition comprend au moins un premier polymère siliconé de viscosité comprise entre 10 et 10 000 cSt, et au moins une deuxième polymère siliconé de viscosité supérieure à celle du premier polymère siliconé et comprise entre 5 000 et 300 000 cSt, lesdites viscosités étant mesurées selon la norme ASTM D-445.

Le deuxième polymère siliconé a de préférence une viscosité comprise entre 5 000 et 300 000 cSt , notamment comprise entre 10 000 et 200 000 cSt, par exemple entre 30 000 et 100 000 cSt.

En particulier, le premier polymère siliconé de viscosité comprise entre 10 et 10 000 cSt, a de préférence une viscosité comprise entre 10 et 5 000 cSt, de préférence entre 10 et 1 000 cSt, de préférence encore entre 10 et 500 cSt, et le deuxième polymère siliconé de viscosité supérieure à celle du premier polymère siliconé comprise entre 5 000 et 300 000 cSt a de préférence un viscosité comprise entre 10 000 et 300 000 cSt.

La proportion massique entre les deux polymères siliconés est avantageusement ajustée selon la viscosité de la deuxième composition que l'on souhaite obtenir, afin d'optimiser la tenue et le non collant du produit cosmétique selon l'invention.

Le polymère de viscosité comprise entre 5 000 et 300 000 cSt peut répondre à la formule (B):

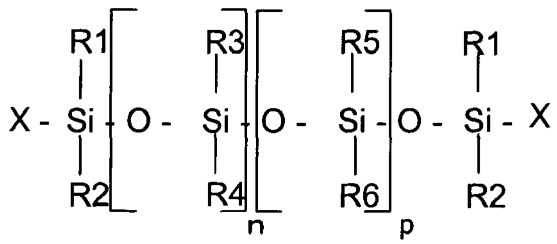

(B)

dans laquelle :

$R_1$, $R_2$, $R_5$ et $R_6$ sont, ensemble ou séparément, un radical alkyle ayant 1 à 6 atomes de carbone, éventuellement substitué par au moins un atome de fluor,
$R_3$ et $R_4$ sont, ensemble ou séparément, un radical alkyle ayant de 1 à 6 atomes de carbone ou un radical aryle,
X est un radical alkyle ayant de 1 à 6 atomes de carbone, un radical hydroxyle, un radical vinyle, allyle ou un radical alkoxyle ayant de 1 à 6 atomes de carbone,
n et p étant choisis de manière à ce que le polymère siliconé a une viscosité à 25°C, mesurée selon la norme ASTM D-445 comprise entre 5 000 et 300 000 cSt.
p est de préférence égal à 0.

Le polymère siliconé de viscosité comprise entre 5 000 et 300 000 cSt peut être choisi parmi les polyalkylsiloxanes, les polyarylsiloxanes, les polyalkylarylsiloxanes et leurs mélanges.

Le polymère siliconé de viscosité comprise entre 5 000 et 300 000 cSt peut être notamment parmi :

- les polydiméthylsiloxanes (PDMS);
- les polydiméthylsiloxanes comportant des groupements alkyle, alcoxy ou phényle, pendant ou en bout de chaîne siliconée, groupements ayant de 2 à 24 atomes de carbone ;
- les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les polyalkylsiloxanes selon l'invention incluent les polydiméthylsiloxanes, les copolymères (polydiméthylsiloxane)(méthylvinylsiloxane), les poly(diméthylsiloxane)(diphényl)siloxanes, les copolymères poly(diméthylsiloxane)(diphényl)(méthylvinylsiloxane), et leurs mélanges

Le polymère siliconé de viscosité comprise entre 5 000 et 300 000 cSt est avantageusement une polydiméthylsiloxane.

Les deux polymères siliconés de viscosité différentes sont avantageusement des polydiméthylsiloxanes.

Selon un des modes de réalisations de l'invention, la seconde composition comprend au moins une première polydiméthylsiloxane de viscosité comprise entre 50 et 5 000 cSt, et au moins une deuxième polydiméthylsiloxane de viscosité comprise entre 10 000 et 300 000 cSt, lesdites viscosité étant mesurées selon la norme ASTM D-445.

Dans ce mode de réalisation, la proportion des deux polydiméthylsiloxanes est avantageusement choisie de telle sorte

que la viscosité de la composition est comprise entre 5 et 25 Pa.s.

Le premier polymère siliconé a par exemple une viscosité mesurée à 25°C selon la norme ASTM D-445 comprise entre 50 et 1 000 cSt, notamment entre 100 et 500 cSt, et le second polymère siliconé a notamment une viscosité mesurée à 25°C selon la norme ASTM D-445 comprise entre 30 000 et 300 000 cSt, notamment entre 30 000 et 250 000 cSt.

Les deux polymères siliconés peuvent être choisis parmi les polymères décrits précédemment.

La deuxième composition contient de préférence moins de 50% en poids d'une huile siliconée ayant une viscosité à 25°C mesurée selon la norme ASTM D-445 inférieure à 50 cSt, notamment inférieure à 20 cSt, notamment inférieure à 10 cSt, notamment inférieure ou égale à 5 cSt. La deuxième composition contient de préférence moins de 40%, de préférence moins de 30%, de préférence moins de 20% en poids, de cette huile siliconée, dont la viscosité peut être supérieure ou égale à 0,01 cSt.

La deuxième composition contient de préférence moins de 10% en poids de pigments et/ou de charges tels que définis ci-après. Notamment, la deuxième composition contient moins de 5% en poids de pigments et/ou de charges.

***Polymère siliconé de très haute viscosité***

La deuxième composition peut comprendre en outre un polymère siliconé de viscosité supérieure à 300 000 cSt, ou un polymère siliconé solide éventuellement fourni sous forme solubilisée dans un solvant.

Ce polymère est avantageusement un polymère non greffé, c'est à dire un polymère obtenu par polymérisation d'au moins un monomère, sans réaction subséquente des chaînes latérales avec un autre composé chimique. Le polymère est de préférence choisi parmi les diméthiconols, les fluorosilicones, les diméthicones et leurs mélanges.

Selon un mode de mise en oeuvre, le polymère siliconé de viscosité supérieure à 300 000 cSt est la diméthiconol commercialisée par Dow Corning sous la référence D2-9085, ou la diméthiconol commercialisée par Dow Corning sous la référence DC 1503. On peut également choisir la diméthiconol commercialisée par Dow Corning sous la référence Q2-1403 ou Q2-1401.

Comme polymère siliconé de viscosité supérieure à 300 000 cSt, on peut citer également SE30 SE33, SE 54 et SE 76 vendus par la société Général Electric, et AK 500000 vendu par la société Waker, et Silbione 70047 V vendu par la société Rhodia.

Les diméthicones selon l'invention incluent les polydiméthylsiloxanes, les copolymères (polydiméthylsiloxane)(méthylvinylsiloxane), les copolymères poly(diméthylsiloxane)(diphényl)(méthylvinylsiloxane), et leurs mélanges.

La quantité de polymère siliconé de viscosité supérieure à 300 000 cSt ou de polymère siliconé solide est de préférence comprise entre 0,1% et 20% préférentiellement 0,1 et 5%.

***Huile volatile***

La deuxième composition peut éventuellement contenir une huile volatile.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm de Hg), et de préférence allant de 1,3 Pa à 13 000 Pa (0,01 à 100 mm de Hg), et préférentiellement allant de 1,3 Pa à 1 300 Pa (0,1 à 10 mm de Hg).

En outre, l'huile volatile a généralement un point d'ébullition, mesuré à pression atmosphérique, allant de 150 °C à 260 °C, et de préférence allant de 170 °C à 250 °C.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.

Par huile siliconée, on entend une huile contenant au moins un atome de silicium, et notamment contenant des groupes Si-O.

L'huile volatile peut être siliconée ou hydrocarbonée.

L'huile volatile siliconée utilisable dans l'invention peut être choisie parmi les huiles siliconées ayant un point éclair allant de 40 °C à 102 °C, de préférence ayant un point éclair supérieur à 55 °C et inférieur ou égal à 95 °C, et préférentiellement allant de 65 °C à 95 °C.

Comme huiles siliconées volatiles utilisables dans l'invention, on peut citer les silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl-cyclotétrasiloxane, le décaméthyl-cyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huiles siliconées volatiles utilisables dans l'invention, on peut citer les silicones décrites dans la demande FR0304259 non publiée.

L'huile volatile hydrocarbonée utilisable dans l'invention peut être choisie parmi les huiles hydrocarbonées ayant un point éclair allant de 40 °C à 102 °C, de préférence allant de 50 °C à 100 °C, et préférentiellement allant de 75 °C à 85 °C.

Comme huile volatile hydrocarbonée, on peut citer les huiles volatiles hydrocarbonées ayant de 8 à 16 atomes de carbone et leurs mélanges, et notamment les alcanes ramifiés en $C_8$-$C_{16}$ comme les iso-alcanes (appelées aussi isoparaffines) en $C_8$-$C_{16}$, l'isododécane, l'isodécane, l'isohexadécane et par exemple les huiles vendues sous les noms commerciaux d'Isopars ou de Permetyls, les esters ramifiés en $C_8$-$C_{16}$ comme le néopentanoate d'isohexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

L'huile volatile représente de préférence de 0,1 à 50 % du poids total de la composition, et mieux de 1 à 30 %, de préférence entre 5 et 15% du poids total de la composition.

***Agent gélifiant***

Selon un mode de réalisation de l'invention, la deuxième composition peut comprendre un agent gélifiant, lequel est un gélifiant polymère ou minéral.

Par "agent gélifiant", on entend un composé qui augmente la viscosité du milieu dans lequel il est incorporé, ou qui rigidifie ledit milieu. L'agent gélifiant selon l'invention n'inclut pas les cires.

Selon un autre mode de mise en oeuvre, l'agent gélifiant peut être notamment,

- une silice pyrogénée éventuellement traitée hydrophobe en surface;
- les argiles éventuellement modifiées comme les hectorites modifiées par un chlorure d'ammonium d'acide gras en $C_{10}$ à $C_{22,}$ comme l'hectorite modifiée par du chlorure de di-stéaryl di-méthyl ammonium et leurs mélanges;
  Comme gélifiant, on peut également également citer les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric.

***Cire***

La deuxième composition peut contenir au moins une cire.

Les cires peuvent être présentes à raison de 2-30% en poids dans la composition et mieux de 5 à 20%, de préférence entre 5 et 15%, de la composition.

On préfère dans le cadre de la présente invention des cires hydrocarbonées linéaires. Leur point de fusion est avantageusement supérieur à 35°C, par exemple supérieur à 55°C, de préférence supérieur à 80°C.
Les cires hydrocarbonées linéaires sont avantageusement choisies parmi les alcanes linéaires substitués, les alcanes linéaires non substitués, les alcènes linéaires non substitués, les alcènes linéaires substitués, un composé non substitué étant uniquement composé de carbone et d'hydrogène. Les substituants mentionnés précédemment ne contenant pas d'atomes de carbone.
Les cires hydrocarbonées linéaires incluent les polymères et copolymères de l'éthylène, de poids moléculaire compris entre 400 et 800, par exemple la Polywax 500 ou Polywax 400 commercialisée par New Phase Technologies.
Les cires hydrocarbonées linéaires incluent les cires de paraffine linéaires, comme les paraffines S&P 206, S&P 173 et S&P 434 de Strahl & Pitsch.

Les cires hydrocarbonées linéaires incluent les alcools linéaires à longue chaîne, comme les produits comprenant un mélange de polyéthylène et d'alcools en comprenant 20 à 50 atomes de carbones, notamment le Performacol 425 ou le Performacol 550 (mélange dans les proportions 20/80) commercialisés par New Phase Technologies.

La deuxième composition contient avantageusement une cire siliconée, telle qu'une diméthicone comprenant des groupements alkyles en bout de chaîne. Ces groupements alkyles ont de préférence plus de 18 atomes de carbones, de préférence entre 20 et 50, de préférence entre 30 et 45 atomes de carbone. Des exemples de cires siliconées sont par exemple

- Les C20-24 alkyl méthicone, C24-28 alkyl diméthicone, C20-24 alkyl diméthicone, C24-28 alkyl diméthicone commercialisées par Archimica Fine Chemicals sous la référence SilCare 41M40, SilCare 41M50, SilCare 41M70 and SilCare 41M80,

- Les stéaryl diméthicone de référence SilCare 41M65 commercialisées par Archimica

ou de référence DC-2503 commercialisée par Dow-Corning

- Les stéaroxytriméthylsilane vendues sous la référence SilCare 1M71 ou DC-580
- Les produits ABIL Wax 9810, 9800, or 2440 de Wacker-Chemie GmbH,
- Les C30-45 alkyl méthicone commercialisées par Dow Corning sous la référence AMS-C30 Wax, de même que les C30-45 alkyl diméthicone commercialisées par General Electric sous la référence SF1642 ou SF-1632.

Selon un mode de réalisation, la composition contient moins de 5% en poids, de préférence moins de 3%, voir moins de 1% en poids de cire.

**Première composition**

La première composition comprend un milieu physiologiquement acceptable, i.e. cosmétiquement ou dermatologiquement acceptable, à savoir contenir un milieu non toxique et susceptible d'être appliqué sur la peau, les phanères ou les lèvres du visage d'êtres humains. Par cosmétiquement acceptable, on entend au sens de l'invention une composition d'aspect, d'odeur et de toucher agréables.

La première composition peut être tout type de composition cosmétique connue de l'homme du métier. De préférence, la première composition est une composition cosmétique ayant de bonnes propriétés de tenue, en particulier de bonne tenue à l'huile.

***Polymère filmogène***

Selon un mode de mise en oeuvre, la première composition selon l'invention comprend avantageusement un polymère filmogène et une phase organique liquide, notamment une phase grasse liquide.

Par polymère "filmogène", on entend un polymère apte à former à lui seul ou en présence d'un agent auxiliaire de filmification, un film continu et adhérent sur un support, notamment sur les matières kératiniques, et de préférence un film cohésif et mieux encore, un film dont la cohésion et les propriétés mécaniques sont telles que ledit film peut être isolé dudit support.

Le polymère filmogène est par exemple l'un des polymères décrits dans la demande FR0450540 déposée le 18 mars 2004, dont le contenu est inclus dans la présente demande par référence.

Par "phase grasse liquide", au sens de la demande, on entend tout milieux non aqueux liquide à température ambiante (25°C) et pression atmosphérique (760 mm de Hg), composé d'un ou plusieurs corps gras liquides à température ambiante, appelés aussi huiles, à l'exception de l'ester d'acide et de polyol, ainsi que des gélifiants et des stabilisants des pigments éventuellement présents dans la composition, lorsqu'ils sont liquides à température ambiante et pression atmosphérique. Cette phase grasse peut contenir une phase grasse liquide volatile et/ou une phase grasse non volatile.

Le polymère filmogène est de préférence un polymère éthylénique non cristallin.

Par polymère "éthylénique", on entend un polymère obtenu par polymérisation de monomères comprenant une insaturation éthylénique.

Le polymère filmogène est de préférence sous la forme d'une dispersion de particules dans une phase grasse liquide.

Selon un premier mode de mise en oeuvre, la première composition comprend une dispersion stable de particules de polymère généralement sphériques d'un ou plusieurs polymères, dans une phase organique liquide physiologiquement acceptable.

Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500nm. Il est toutefois possible d'obtenir des tailles de particules de polymère allant jusqu'à 1$\mu$m.

Ces dispersions de particules de polymère sont notamment celles décrites dans la demande EP749747 dont le contenu est incorporé dans la présente demande par référence.

De préférence, les particules de polymères en dispersion sont insolubles dans les alcools hydrosolubles tels que, par exemple, l'éthanol.

Les polymères en dispersion utilisables dans la première composition de l'invention ont de préférence un poids moléculaire de l'ordre de 2000 à 10 000 000 g/mol, et une Tg de -100°C à 300°C et mieux de -50° à 100°C, de préférence de -10°C à 50°C.

Lorsque le polymère présente une température de transition vitreuse trop élevée pour l'application souhaitée,

on peut lui associer un plastifiant de manière à abaisser cette température du mélange utilisé.

Il est possible d'utiliser des polymères de préférence ayant une Tg basse, inférieure ou égale à la température de la peau et notamment inférieure ou égale à 40°C et notamment allant de —10° à 30°C.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères acryliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en $C_1$-$C_{20,}$ de préférence en $C_1$-$C_{8,}$ les (méth)acrylates d'aryle, en particulier d'aryle en $C_6$-$C_{10,}$ les (méth)acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et de lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de benzyle ou de phényle.

Les esters de l'acide (méth)acrylique particulièrement préférés sont les (méth)acrylates d'alkyle.

Comme polymère radicalaire, on utilise de préférence les copolymères d'acide (méth)acrylique et de (méth) acrylate d'alkyle, notamment d'alkyle en $C_1$-$C_4$. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth) acrylamides, en particulier d'alkyle en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères acryliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyle, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques utilisables, on peut encore citer :

- la N-vinylpyrrolidone, la vinylcaprolactame, les vinyl N-alkyl($C_1$-$C_6$) pyrroles, les vinyl-oxazoles, les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phtalate de diallyle.

De façon non limitative, les polymères en dispersion de l'invention peuvent être choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides, polyesters à chaîne grasse, alkydes ; polymères ou copolymères acryliques et/ou vinyliques ; copolymères acryliques-silicone ; polyacrylamides ; polymères siliconés comme les polyuréthanes ou acryliques siliconés, polymères fluorés et leurs mélanges.

Le ou les polymères en dispersion dans la phase liquide organique peuvent représenter en matière sèche de 2 à 40% du poids de la composition, de préférence de 5 à 40 %, de préférence de 5 à 35 % et mieux de 8 à 30%.

La première composition comprend avantageusement un agent stabilisant qui peut être un polymère séquencé, un polymère greffé, et/ou un polymère statistique, seul ou en mélange. La stabilisation peut être effectuée par tout moyen connu, et en particulier par ajout direct du polymère séquencé, polymère greffé et/ou polymère statistique, lors

de la polymérisation. Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylènebutylène. Parmi ces polymères, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'Kraton' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS). Les polymères sont généralement appelés des copolymères de diènes hydrogénés ou non.

Le stabilisant est de préférence également présent dans le mélange avant polymérisation du polymère. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Dans ce mode de mise en oeuvre, la phase grasse liquide de la première composition peut être constituée de toute huile cosmétiquement ou dermatologiquement acceptable, et de façon générale physiologiquement acceptable, notamment choisie parmi les huiles d'origine minérale, végétale ou synthétique, carbonées, hydrocarbonées, fluorées et/ou siliconées, seules ou en mélange dans la mesure où elles forment un mélange homogène et stable macroscopiquement et où elles sont compatibles avec l'utilisation envisagée.

La phase grasse liquide totale de la composition peut représenter de 5 % à 90 % du poids total de la composition et de préférence de 20 à 85 %. Avantageusement, elle représente au moins 30 % du poids total de la composition. De préférence, cette phase grasse contient au moins une huile volatile.

Selon l'invention, on utilise avantageusement une ou plusieurs huiles volatiles.

Ces huiles peuvent être des huiles hydrocarbonées ou des huiles siliconées comportant éventuellement des groupements alkyle ou alkoxy pendants ou en bout de chaîne siliconée.

Comme huile de silicone volatile utilisable dans l'invention, on peut citer les silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme autre huile volatile utilisable dans l'invention, on préfère notamment les huiles isoalcanes (appelées aussi isoparaffines) en $C_8$-$C_{16}$ comme l'isododécane, l'isodécane, l'isohexadécane et par exemple les huiles vendus sous les noms commerciaux d'ISOPAR, de PERMETHYL et notamment l'isododécane (PERMETHYL 99 A).

Les huiles volatiles représentent notamment de 5 à 85 % du poids total de la composition, et mieux de 20 à 75 %.

Solvant de synthèse des particules de polymère

La dispersion de polymère peut être fabriquée comme décrit dans le document EP-A-749747.

On prépare un mélange comprenant les monomères initiaux ainsi qu'un amorceur radicalaire. Ce mélange est dissous dans un solvant appelé, dans la suite de la présente description, "solvant de synthèse". Lorsque la phase grasse est une huile non volatile, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit un solvant de synthèse tel que les monomères initiaux, et l'amorceur radicalaire, y sont solubles, et les particules de polymère obtenues y sont insolubles afin qu'elles y précipitent lors de leur formation. En particulier, on peut choisir le solvant de synthèse parmi les alcanes tels que l'heptane, l'isododécane ou le cyclohexane.

Lorsque la phase grasse choisie est une huile volatile, on peut directement effectuer la polymérisation dans ladite huile qui joue donc également le rôle de solvant de synthèse. Les monomères doivent également y être solubles, ainsi que l'amorceur radicalaire, et le polymère obtenu doit y être insoluble.

Les monomères sont de préférence présents dans le solvant de synthèse, avant polymérisation, à raison de 5-20% en poids du mélange réactionnel. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

La phase volatile de la composition peut être constituée par le solvant de synthèse des particules de polymères

dispersées.

Huile non volatile de la phase grasse

La phase grasse contient de préférence au moins une huile non volatile apolaire ou peu polaire.

Comme huile non volatile peu polaire utilisable dans l'invention, on peut citer les apolaires ou, notamment les huiles comportant une chaîne alkyle notamment en $C_3$-$C_{40}$. Comme exemple d'huile apolaires ou peu polaires, on peut citer :

- les hydrocarbures linéaires ou ramifiés tels que les huiles de paraffine, de vaseline et de naphtalène légères, le polyisobutène hydrogéné ;
- les huiles hydrocarbonées d'origine animale comme le squalène ;
- les huiles hydrocarbonées végétales telles que les triglycérides liquides d'acides gras d'au moins 10 atomes de carbone;
- les esters et les éthers de synthèse notamment d'acides gras comme les huiles de formule $R_1CO(O)_xR_2$ dans laquelle $R_1$ représente le reste d'un acide comportant de 2 à 29 atomes de carbone avec x valant 0 ou 1 et $R_2$ représente une chaîne hydrocarbonée contenant de 3 à 30 atomes de carbone comme par exemple l'acétyl-citrate de tributyle, l'érucate d'oléyle, le béhénate d'octyl-2 dodécyle, le citrate de tri-iso-arachidyle, le stéaroyl-stéarate d'isocétyle ou d'octyldodécanyle, l'acétate de n-propyle, le tri-méllitate de tri-décyle, le dodécane di-oléate ou le stéarate de di-iso-cétyle, le propionate d'arachidyle, le dibutyl phtalate, le carbonate de propylène, le pentanoate d'octyldodécyle ; les esters de polyol comme la vitamine F, l'isostéarate de sorbitane, le triisostéarate de glycérine ou de diglycérine ;
- les huiles siliconées telles que les polydiméthylsiloxanes (PDMS), comportant éventuellement une chaîne alkyle ou alcoxy en $C_3$-$C_{40}$ ou une chaîne phénylée telle que les phényltriméthicones, les polyalkylméthylsiloxanes, éventuellement fluorés comme les polyméthyltrifluoro-propyldiméthylsiloxanes, ou par des groupements fonctionnels tels que des groupements hydroxyle, thiol et/ou amine ; les polysiloxanes modifiés par des acides gras, des alcools gras ou des polyoxyalkylènes,
- les huiles fluorées;
- leurs mélanges.

Ces huiles apolaires ou peu polaires non volatiles peuvent représenter de 0,1 à 20 % du poids total de la composition, mieux de 0,5 à 10 %.et mieux encore de 1 à 5% du poids total de la composition.

De préférence, l'huile non volatile est apolaire. Elle est avantageusement choisie parmi les hydrocarbures notamment les alcanes comme le polyisobutène hydrogéné.

La phase grasse peut également contenir une huile polaire choisie parmi les esters d'acide gras de 7 à 29 atomes de carbone comme le malate de diisostéaryle, le palmitate d'isopropyle, l'adipate de diisopropyle, les triglycérides des acides caprylique/caprique comme ceux vendus par la société Stearineries Dubois ou ceux vendus sous les dénominations Miglyol 810, 812 et 818 par la société Dynamit Nobel, l'huile de beurre de karité, le myristate d'isopropyle, le stéarate de butyle, le laurate d'hexyle, l'adipate de diisopropyle, l'isononate d'isononyle, le laurate de 2-hexyl-décyle, le palmitate de 2-octyl-décyle, le myristate ou le lactate de 2-octyl-dodécyle, le succinate de 2-diéthyl-hexyle, le palmitate d'éthyl-2hexyle, le stéarate d'octyl-2-dodécyle, l'huile de ricin ; les esters d'acide lanolique, d'acide laurique, d'acide stéarique ; les alcools gras supérieurs (de 7 à 29 atomes de carbone) tels que l'alcool stéarylique, l'alcool linoléique ou linolénique, l'alcool isostéarique, l'octyl 2-dodécanol, le décanol, le dodécanol, l'octadécanol ou l'alcool oléique ; les acides gras supérieurs (de 7 à 29 atomes de carbone) tels que l'acide myristique, l'acide palmitique, l'acide stéarique, l'acide béhénique, l'acide oléique, l'acide linoléique, l'acide linolénique ou l'acide isostéarique ; leurs mélanges.

Ces huiles polaires non volatiles peuvent représenter de 0,1 à 10 % du poids total de la composition et mieux de 1 à 5 %.

Selon un mode particulier de l'invention, la phase grasse liquide contient au moins une huile volatile apolaire et au moins une huile non volatile apolaire.

Dans ce mode de mise en oeuvre, la première composition contient avantageusement un agent dispersant des charges et/ou des pigments.

En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans la composition de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy-12-stéarique sont de préférence destinés à un milieu hydrocarboné ou fluoré, alors que les mélanges de diméthylsiloxane oxyéthylène/oxypropyléné sont de préférence destinés à un milieu siliconé.

Selon un autre mode de mise en oeuvre, la première composition selon l'invention peut comprendre à titre de polymère filmogène une dispersion de particules, de préférence solides, d'un polymère éthylénique greffé dans une phase grasse liquide.

Par polymère "greffé", on entend un polymère ayant un squelette comprenant au moins une chaîne latérale pendante ou située en bout de chaîne, et de préférence pendante.

Avantageusement, le polymère éthylénique greffé comprend un squelette éthylénique insoluble dans ladite phase grasse liquide, et des chaînes latérales liées de manière covalente audit squelette et solubles dans la phase grasse liquide.

Le polymère éthylénique greffé est notamment un polymère non réticulé. En particulier, le polymère est obtenu par polymérisation de monomères comprenant un seul groupement polymérisable.

Selon un mode de réalisation de l'invention, le polymère éthylénique greffé est un polymère acrylique greffé.

Le polymère éthylénique greffé est notamment susceptible d'être obtenu par polymérisation radicalaire dans un milieu organique de polymérisation :

- d'au moins un monomère éthylénique, en particulier d'au moins un monomère acrylique et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

La phase grasse liquide peut contenir le milieu organique de polymérisation du polymère éthylénique greffé.

Le milieu organique liquide de dispersion, correspondant au milieu dans lequel est fourni le polymère greffé, peut être identique au milieu de polymérisation.

Toutefois, le milieu de polymérisation peut être substitué en tout ou partie par un autre milieu organique liquide. Cet autre milieu organique liquide peut être ajouté, après polymérisation, au milieu de polymérisation. Ce dernier est ensuite évaporé en tout ou partie.

La phase grasse liquide peut contenir des composés liquides organiques autres que ceux présents dans le milieu de dispersion. Ces autres composés sont choisis de manière à ce que le polymère greffé reste à l'état de dispersion dans la phase grasse liquide.

Le milieu liquide organique de dispersion est présent dans la phase grasse liquide de la composition selon l'invention du fait de l'introduction dans la composition de la dispersion de polymère greffé obtenue.

La phase grasse liquide peut être une phase grasse liquide non siliconée.

On entend par "phase grasse liquide non siliconée" une phase grasse comprenant un ou plusieurs composés organiques liquides ou huiles non siliconé(e)s, tels que ceux cités précédemment, lesdits composés non siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids), ou encore de 65 % à 100 % en poids (par exemple de 65 à 95 % en poids), par rapport au poids total de la phase grasse liquide.

Les composés organiques liquides non siliconés peuvent être notamment choisis parmi :

- les composés organiques liquides non siliconés ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$ ; et
- leurs mélanges.

Ladite phase grasse liquide non siliconée peut donc éventuellement comprendre des composés organiques liquide ou huiles siliconé(e)s, tels que ceux cités précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total de la phase grasse liquide.

Selon un mode particulier de réalisation de l'invention, la phase grasse liquide non siliconée ne contient pas de composés organiques liquides ou huiles siliconé(e)s.

Lorsque la phase grasse liquide est une phase grasse liquide non siliconée, les macromonères présents dans le polymère greffé sont avantageusement des macromonomères carbonés tels que décrits ci-après.

En particulier, lorsque la phase grasse liquide est une phase grasse liquide non siliconée, le polymère greffé présent dans la composition est avantageusement un polymère greffé non siliconé.

Par polymère greffé non siliconé, on entend un polymère greffé contenant majoritairement un macromonomère carboné et contenant éventuellement au plus 7 % en poids, de préférence au plus 5 % en poids, voire est exempt, de macromonomère siliconé.

Le choix des monomères constituant le squelette du polymère, des macromonomères, le poids moléculaire du polymère, la proportion des monomères et des macromonomères peut être fait en fonction du milieu organique liquide de dispersion de manière à obtenir avantageusement une dispersion de particules de polymères greffés en particulier une dispersion stable, ce choix pouvant être effectué par l'homme du métier.

Par "dispersion stable", on entend une dispersion qui n'est pas susceptible de former de dépôt solide ou de déphasage liquide/solide notamment après une centrifugation, par exemple, à 4000 tours/minute pendant 15 minutes.

Selon l'invention, on entend par "polymère acrylique greffé " un polymère susceptible d'être obtenu par polymérisation radicalaire :

- d'un ou plusieurs monomère(s) acrylique(s), et éventuellement d'un ou plusieurs monomère(s) additionnel(s) vinylique(s) non acrylique(s),
- avec un ou plusieurs macromonomère(s), dans un milieu organique de polymérisation.

Avantageusement, les monomères acryliques représentent de 50 à 100 % en poids, de préférence de 55 à 100 % en poids (notamment de 55 à 95 % en poids), préférentiellement de 60 à 100 % en poids (notamment de 60 à 90 % en poids) du mélange monomères acryliques + monomères vinyliques non acryliques éventuels.

De préférence, les monomères acryliques sont choisis parmi les monomères dont l'homopolymère est insoluble dans le milieu de dispersion considéré, c'est-à-dire que l'homopolymère est sous forme solide (ou non dissous) à une concentration supérieure ou égale à 5% en poids à température ambiante (20°C) dans ledit milieu de dispersion.

a) Macromonomères :

Selon l'invention, on entend par "macromonomère ayant un groupe terminal polymérisable" tout polymère comportant sur une seule de ses extrémités un groupe terminal polymérisable apte à réagir lors de la réaction de polymérisation avec les monomères acryliques et éventuellement les monomères vinyliques non acryliques additionnels constituant le squelette. Le macromonomère permet de former les chaînes latérales du polymère acrylique greffé. Le groupe polymérisable du macromonomère peut être avantageusement un groupe à insaturation éthylénique susceptible de se polymériser par voie radicalaire avec les monomères constituant le squelette.

Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.

De préférence, le macromonomère est choisi parmi les macromonomères dont l'homopolymère est soluble dans le milieu de dispersion considéré, c'est-à-dire complètement dissous à une concentration supérieure ou égale à 5 % en poids et à température ambiante dans ledit milieu de dispersion.

Ainsi, le polymère acrylique greffé comprend un squelette (ou chaîne principale) constitué par un enchaînement de motifs acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale.

Le squelette (ou chaîne principale) est insoluble dans le milieu de dispersionconsidéré alors que les chaînes latérales (ou greffons) sont solubles dans ledit milieu de dispersion.

b) Les monomères:

Par "monomère acryliques", on entend dans la présente demande des monomères choisis parmi l'acide (méth) acrylique, les esters de l'acide (méth)acrylique (appelés également les (méth)acrylates), les amides de l'acide (méthacrylique) (appelés également les (méth)acrylamides).

Parmi les monomères acryliques, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; et leurs sels ; et leurs mélanges.

De préférence, les monomères acryliques sont choisis parmi l'acrylate de méthyle, l'acrylate de méthoxyéthyle, le méthacrylate de méthyle, le méthacrylate de 2-hydroxyéthyle, l'acide acrylique, le méthacrylate de diméthylaminoéthyle, et leurs mélanges.

Avantageusement, les monomères acryliques présents dans le polymère greffés comprennent au moins l'acide

(méth)acrylique et au moins un monomère choisi parmi les (méth)acrylates et les (méth)acrylamides décrits précédemment aux points (i) et (ii). De préférence, les monomères acryliques comprennent au moins l'acide (méth)acrylique et au moins un monomère choisi parmi les (méth)acrylates d'alkyle en $C_1$-$C_3$. L'acide (méth)acrylique peut être présent en une teneur d'au moins 5 % en poids, par rapport au poids total du polymère, notamment allant de 5 % à 80 % en poids, de préférence d'au moins 10 % en poids, notamment allant de 10 % en poids à 70 % en poids, préférentiellement d'au moins 15 % en poids, notamment allant de 15 % à 60 % en poids.

Comme monomère acrylique principal, on peut utiliser l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'iso-propyle, et leurs mélanges.
On préfère tout particulièrement l'acrylate de méthyle, le méthacrylate de méthyle, le méthacrylate d'éthyle.

Les monomères acryliques additionnels peuvent être choisis parmi :

- l'acide (méth)acrylique et ses sels,
- les (méth)acrylates de formule (1) et leurs sels :

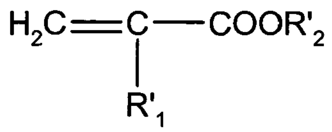

(I)

dans laquelle :

- R'$_1$ désigne un atome d'hydrogène ou un groupe méthyle ;
- R'$_2$ représente
- un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi —OH, les atomes d'halogène (F, Cl, Br, I) et -NR'R" avec R' et R" identiques ou différents choisis parmi les alkyles linéaires ou ramifiés en $C_1$-$C_3$ ;
    - un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;
- et leurs mélanges.

A titre d'exemples de R'$_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

Parmi ces monomères acryliques additionnels, on peut tout particulièrement citer l'acide (méth)acrylique, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle, leurs sels, et leurs mélanges.
On préfère tout particulièrement l'acide acrylique, l'acide méthylacrylique.

Les macromonomères comportent à une des extrémités de la chaîne un groupe terminal polymérisable apte à réagir au cours de la polymérisation avec les monomères acryliques et éventuellement les monomères vinyliques additionnels, pour former les chaînes latérales du polymère éthylénique greffé. Ledit groupe terminal polymérisable peut être en particulier un groupe vinyle ou (méth)acrylate (ou (méth)acryloxy), et de préférence un groupe (méth)acrylate.

Les macromonomères sont choisis préférentiellement parmi les macromonomères dont l'homopolymère a une température de transition vitreuse (Tg) inférieure ou égale à 25°C, notamment allant de - 100°C à 25°C, de préférence allant de - 80°C à 0°C.

Les macromonomères ont une masse moléculaire moyenne en poids supérieure ou égale à 200, de préférence supérieure ou égale à 300, préférentiellement supérieure ou égale à 500, et plus préférentiellement supérieure à 600. De préférence, les macromonomères ont une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.

Dans la présente demande, les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

Comme macromonomères carbonés, on peut en particulier citer :

- (i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer en particulier : les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth) acrylate.

De tels macromonomères sont notamment décrits dans les brevets EP895467 et EP96459 et dans l'article Gillman K.F., Polymer Letters, Vol 5, page 477-481 (1967).
On peut en particulier citer les macromonomères à base de poly(acrylate d'éthyl-2-hexyle) ou de poly(acrylate de dodécyle) à extrémité mono(méth)acrylate.

- (ii) les polyoléfines ayant un groupe terminal à insaturation éthylénique , en particulier ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;

De tels macromonomères sont en particulier décrits dans US5625005 qui mentionne des macromonomères éthylène/butylène et éthylène/propylène à groupement terminal réactif (méth)acrylate.
On peut en particulier citer le méthacrylate de poly(éthylène/butylène), tel que celui commercialisé sous la dénomination Kraton Liquid L-1253 par Kraton Polymers.
De préférence, le polymère greffé a une masse moléculaire moyenne en poids (Mw) comprise entre 10 000 et 300 000, notamment entre 20 000 et 200 000, mieux encore entre 25 000 et 150 000.
De préférence, la dispersion de particules de polymère greffé présente un taux de matière sèche (ou extrait sec) en polymère pouvant aller de 40 % à 70 % en poids de matière sèche, notamment allant de 45 % à 65 % en poids.
Le polymère greffé peut être présent dans la composition selon l'invention en une teneur en matière sèche (ou matière active) allant de 1 à 70% en poids par rapport au poids total de la composition, mieux de 5 à 60% en poids, de préférence allant de 6 à 45% et mieux allant de 8 à 40% en poids.
Le polymère éthylénique greffé est décrit plus en détail dans la demande FR 0450540 déposée le 18 mars 2004 non publiée dont le contenu est inclus dans la présente demande par référence.

***Plastifiant***

Dans le cadre de la présente invention, on peut associer au polymère filmogène un plastifiant du polymère particulier, de manière à abaisser la Tg du film de polymère et améliorer l'adhérence du film de polymère sur son support, en particulier les matières kératiniques. Le plastifiant abaisse notamment la température de transition vitreuse du polymère d'au moins 1, 2, 3 ou 4 °C, de préférence de 5°C à 20°C. Dans un mode de réalisation préféré, le plastifiant abaisse notamment la température de transition vitreuse du polymère d'au moins 1, 2, 3 ou 4 °C, de préférence de 5°C à 20°C, lorsque le plastifiant représente au plus 10% en poids du polymère.
Selon un mode de mise en oeuvre, le plastifiant du polymère est choisi parmi les plastifiants dont le paramètre de solubilité $\delta_h$ est compris entre 5,5 et 11, de préférence entre 5,9 et 11, de préférence entre 7 et 10,5, de préférence entre 9 et 10, de préférence encore entre 8 et 10 $(J/cm^3)^{½}$.
Le paramètre de solubilité $\delta_P$ du plastifiant est de préférence compris entre 1,5 et 4,5, de préférence entre 1,5 et 4, de préférence entre 1,5 et 3,5, de préférence encore entre 2 et 3 $(J/cm^3)^{½}$.
La définition des paramètres de solubilité selon HANSEN est bien connue de l'homme du métier, et notamment décrite dans l'article de C. M. HANSEN : "The three dimensional solubility parameters" J. Paint Technol. 39, 105 (1967). Ces paramètres sont aussi décrits dans le document JP-A-08-109121 de KAO et le document de D.W. Van KREVELEN "Properties of polymers" (1990), p. 190.
Il est à la portée de l'homme du métier de déterminer les quantités de chaque plastifiant pour obtenir un mélange de plastifiants répondant aux relations ci-dessus.
Dans ce mode de réalisation, le plastifiant peu être choisi parmi les esters d'au moins un acide carboxylique comprenant 1 à 7 atomes de carbones et d'un polyol comprenant au moins 4 groupes hydroxyles.
Le polyol peut être un mono- ou un polysaccharide comprenant un à 10 oses, de préférence de un à 4, de

préférence encore un ou deux oses. Le polyol peut être choisi parmi l'érythritol, le xylitol, le sorbitol, le glucose, et le saccharose.

L'ester peut être obtenu par copolymérisation de deux esters selon l'invention, en particulier par copolymérisation i) d'un saccharose substitué par des groupements benzoyle et ii) d'un saccharose substitué par des groupements acétyle et/ou isobutyryle.

L'acide est de préférence un acide monocarboxylique choisi en particulier parmi les acides comprenant 1 à 7 atomes de carbones, de préférence 1 à 5 atomes de carbone, par exemple les acides acétique, n-propanoïque, isopropanoïque, n-butanoïque, isobutanoïque, tertiobutanoïque, n-pentanoïque et benzoïque.

L'ester peut être obtenu à partir d'au moins deux acides monocarboxyliques différents. Selon un mode de mise en oeuvre, l'acide est un acide linéaire ou ramifié non substitué.

L'acide est de préférence choisi parmi l'acide acétique, l'acide isobutyrique et l'acide benzoïque.

Selon un mode de mise en oeuvre préféré, l'ester est le di-acétate-hexa-(2-méthylpropanoate) de saccharose.

Selon un autre mode de mise en oeuvre, le plastifiant selon l'invention est choisi parmi les esters des acides polycarboxyliques aliphatiques ou aromatiques et des alcools aliphatiques ou aromatiques comprenant de 1 à 10 atomes de carbone.

Parmi les citrates, on peut citer l'acétyl-citrate de tributyle, l'acétyl-citrate de triéthyle, l'acétyl-citrate de triéthylhexyle, l'acétyl-citrate de trihexyle, le butyroyl- citrate trihexyle, le citrate d'isodécyle, le citrate d'isopropyle, le citrate de tributyle et le citrate de triéthylhexyle. Selon un mode de mise en oeuvre, l'acide carboxylique n'est pas le citrate d'acétyl tributyle.

Parmi les adipates, on peut citer l'adipate de dibutyle et l'adipate de diéthyl-2-hexyle. Selon un mode de mise en oeuvre, le plastifiant selon l'invention n'est pas l'adipate de diisopropyle.

Parmi les sébaçates, on peut citer le sébaçate de dibutyle, le sébaçate de diéthylhexyle, le sébaçate de diéthyle et le sébaçate de diisopropyle.

Parmi les succinates, on peut citer le succinate de diéthylhexyle et le succinate de diéthyle.

Le plastifiant ne comprend de préférence aucun groupe polaire, en particulier aucun groupe hydroxyle. Les "groupes polaires" sont par exemple des groupes polaires ioniques ou non ioniques choisis parmi -COOH ; -OH ; oxyde d'éthylène; oxyde de propylène; $-PO_4$ ; -NHR ; - $NR_1R_2$ avec $R_1$, $R_2$ formant éventuellement un cycle et représentant un radical alkyle ou alkoxy linéaire ou ramifié en $C_1$ à $C_{20}$.

Le plastifiant est avantageusement présent en une quantité comprise entre 0,1 et 25% en poids, de préférence entre 0,5 et 15% en poids, de préférence encore entre 3 et 15% en poids.

Le rapport massique entre le polymère filmogène et le plastifiant est avantageusement compris entre 0,5 et 100, de préférence entre 1 et 50, de préférence entre 1 et 10, de préférence encore entre 1 à 5.

La première composition peut être notamment l'une des compositions décrites dans la demande FR0350034 déposée le 25/02/2003 dont le contenu est incorporé dans la présente demande par référence.

*Cire*

La première composition du produit selon l'invention peut, de plus, comprendre au moins une cire.

Une cire, au sens de la présente invention, est un composé gras lipophile, solide à température ambiante (25°C), à changement d'état solide/liquide réversible, ayant une température de fusion supérieure à 40°C pouvant aller jusqu'à 200° C, et présentant à l'état solide une organisation cristalline anisotrope.

On préfère dans le cadre de la présente invention des cires hydrocarbonées linéaires. Leur point de fusion est avantageusement supérieur à 35°C, par exemple supérieur à 55°C, de préférence supérieur à 80°C.

Les cires hydrocarbonées linéaires sont avantageusement choisies parmi les alcanes linéaires substitués, les alcanes linéaires non substitués, les alcènes linéaires non substitués, les alcènes linéaires substitués, un composé non substitué étant uniquement composé de carbone et d'hydrogène. Les substituants mentionnés précédemment ne contenant pas d'atomes de carbone.

Les cires hydrocarbonées linéaires incluent les polymères et copolymères de l'éthylène, de poids moléculaire compris entre 400 et 800, par exemple la Polywax 500 ou Polywax 400 commercialisée par New Phase Technologies.

Les cires hydrocarbonées linéaires incluent les cires de paraffine linéaires, comme les paraffines S&P 206, S&P 173 et S&P 434 de Strahl & Pitsch.

Les cires hydrocarbonées linéaires incluent les alcools linéaires à longue chaîne, comme les produits comprenant un mélange de polyéthylène et d'alcools en comprenant 20 à 50 atomes de carbones, notamment le Performacol 425 ou le Performacol 550 (mélange dans les proportions 20/80) commercialisés par New Phase Technologies

Les cires peuvent être présentes à raison de 2-30% en poids dans la composition et mieux de 5 à 20%, de préférence entre 5 et 15%, en vue de ne pas trop diminuer la brillance de la composition et du film déposé sur les lèvres et/ou la peau.

### *Agent de coloration*

La première et/ou la deuxième composition du produit cosmétique selon l'invention peut contenir un agent de coloration qui peut être choisi parmi les colorants hydrosolubles ou liposolubles, les pigments, les nacres et leurs mélanges.

Par « pigments », il faut comprendre des particules blanches ou colorées, minérales ou organiques, insolubles dans la phase organique liquide, destinées à colorer et/ou opacifier la première composition.

Par « nacres », il faut comprendre des particules irisées, notamment produites par certains mollusques dans leur coquille ou bien synthétisées, insolubles dans le milieu de la première composition.

Par « colorants », il faut comprendre des composés généralement organiques solubles dans les corps gras comme les huiles ou dans une phase hydroalcoolique.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.

Les colorants hydrosolubles sont par exemple le jus de betterave, le bleu de méthylène, le caramel.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de zinc, de fer (noir, jaune, brun ou rouge) ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium ou aluminium dont celles soumises à une certification par la Food and Drug Administration (FDA) (exemple D&C

ou FD&C) et ceux exempts de la certification FDA comme les laques à base de carmin de cochenille.

Les nacres ou pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. On peut ainsi utiliser des pigments à propriétés goniochromatiques, et/ou des pigments à effet métallique tel que décrit dans la demande déposée pour le numéro FR 0209246 dont le contenu est incorporé par référence dans la présente demande.

De manière générale, les agents de coloration représentent de 0,001 à 60 %, et mieux de 0,01 à 50 %, et mieux encore, de 0,1 à 40 % du poids total de chaque première et seconde composition.

### *Charges*

La composition selon l'invention peut comprendre au moins une charge, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques

ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La deuxième composition comprend de préférence moins de 15% d'agent de coloration et/ou de charges, de préférence encore moins 10%, de préférence encore moins de 5% en poids.

### *Additifs*

La première et/ou la deuxième composition peut comprendre, en outre, tout autre additif usuellement utilisé dans de telles compositions, tel que des actifs, de l'eau, des antioxydants, des parfums, des tensio-actifs, des conservateurs et des huiles essentielles.

Comme actifs cosmétiques, dermatologiques, hygiéniques ou pharmaceutiques, utilisables dans la composition

de l'invention, on peut citer les hydratants, vitamines, acides gras essentiels, sphingolipides, filtres solaires. Ces actifs sont utilisés en quantité habituelle pour l'homme de l'art et notamment à des concentrations de 0 à 20 % et notamment de 0,001 à 15 % du poids total de la composition.

Bien entendu l'homme du métier veillera à choisir ce ou ces éventuels composés complémentaires, et/ou leur quantité, de manière telle que les propriétés avantageuses de la composition selon l'invention ne soient pas ou substantiellement pas, altérées par l'adjonction envisagée.

***Galéniques***

Un produit de maquillage des lèvres selon l'invention comprend de préférence une première composition sous la forme d'un stick ou sous forme liquide, la deuxième composition étant par exemple sous forme fluide conditionné dans un tube. Le produit selon l'invention comprend deux (ou plusieurs) compositions physiologiquement acceptables conditionnées séparément ou ensemble dans un même article de conditionnement ou dans deux (ou plusieurs) articles de conditionnement séparés ou distincts.

De préférence, ces compositions sont conditionnées séparément.

L'invention a aussi pour objet un kit de maquillage contenant un produit cosmétique de maquillage tel que défini précédemment, dans lequel les différentes compositions sont conditionnées séparément et sont accompagnées de moyens d'application appropriés ou au doigt. Ces moyens peuvent être des pinceaux, des brosses, des stylos, des crayons, des feutres, des plumes, des éponges, des tubes et /ou des embouts mousse. Selon un mode de mise en oeuvre, la première composition est fluide et appliquée à l'aide d'un embout mousse, tandis que la deuxième composition est fluide et appliquée à l'aide d'un tube. Selon un autre mode de mise en oeuvre, la première composition est un stick, tandis que la deuxième composition est fluide et appliquée à l'aide d'un tube. Selon un autre mode de mise en oeuvre, la première composition est fluide et la deuxième composition est sous la forme d'un stick. Selon un autre mode de mise en oeuvre, les deux compositions sont sous la forme d'un stick.

L'invention a encore pour objet un procédé de maquillage de la peau et/ou des lèvres et/ou des phanères, consistant à appliquer sur la peau, les lèvres et/ou les phanères une première couche d'une première composition comprenant un milieu physiologiquement acceptable, puis à appliquer, sur tout ou partie de la première couche, une seconde couche d'une seconde composition.

L'invention a aussi pour objet un support maquillé comprenant une première couche d'une première composition comprenant un milieu physiologiquement acceptable et une seconde couche d'une seconde composition déposée sur tout ou partie de la première couche, la seconde composition comprenant au moins un polymère siliconé de viscosité mesurée selon la norme ASTM D-445 comprise entre 10 et 10 000 cSt.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, ou sous la forme de coupelle utilisable par contact direct ou à l'éponge. En particulier, elles trouvent une application en tant que fond de teint coulé, fard à joues ou à paupières coulé, rouge à lèvres, base ou baume de soin pour les lèvres, produit anti-cernes. Elles peuvent aussi se présenter sous forme d'une pâte souple ou encore de gel, de crème plus ou moins fluide, ou de liquide conditionnés dans un tube. Elles peuvent alors constituées des fonds de teint ou des rouges à lèvres, des produits solaires ou de coloration de la peau.

Ces compositions à application topique peuvent constituer notamment une composition cosmétique, dermatologique, hygiénique ou pharmaceutique de protection, de traitement ou de soin pour le visage, pour le cou, pour les mains ou pour le corps (par exemple crème de soin, huile solaire, gel corporel), une composition de maquillage (par exemple gel de maquillage, crème, stick) ou une composition de bronzage artificiel ou de protection de la peau.

La composition selon l'invention peut se présenter sous la forme d'une composition dermatologique ou de soin de la peau et/ou des phanères ou sous forme d'une composition de protection solaire, d'hygiène corporelle, notamment sous forme de déodorant. Elle se présente alors notamment sous forme non colorée. Elle peut alors être utilisées comme base de soin pour la peau, les phanères ou les lèvres (baumes à lèvres, protégeant les lèvres du froid et/ou du soleil et/ou du vent, crème de soin pour la peau, les ongles ou les cheveux).

Les compositions selon l'invention sont de préférence des rouges à lèvres sous la forme d'un stick ou sous forme fluide.

Chaque composition du produit de maquillage bicouche selon l'invention peut se présenter sous toute forme galénique normalement utilisée pour une application topique et notamment sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules, les vésicules étant situés à l'interface huile/eau, ou d'une poudre. Chaque composition peut être fluide ou solide.

Avantageusement, la première ou la seconde composition, ou les deux, sont à phase grasse continue et de préférence sous forme anhydre et peuvent contenir moins de 5% d'eau, et encore mieux moins de 1% d'eau par rapport au poids total de la première ou seconde composition. En particulier, le produit de maquillage bicouche entier est sous

forme anhydre.

Chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon.

De préférence, la composition qui est appliquée en première couche est sous forme solide, ce qui permet une application plus pratique, une meilleure stabilité dans le temps et en température de la composition et permet un tracé précis du maquillage, ce qui est hautement souhaitable dans le cas d'un rouge à lèvres ou d'un eyeliner.

Le produit est en particulier un rouge à lèvres.

De préférence, la première et/ou la seconde composition est sous forme solide.

Avantageusement, la couche du dessus présente des propriétés de soin, de brillance et de transparence.

L'invention a encore pour objet un produit à lèvres, un fond de teint, un tatouage, un fard à joues ou à paupières contenant une première et une seconde compositions telles que décrites précédemment.

Les compositions de l'invention peuvent être obtenues par chauffage des différents constituants à la température de fusion des cires les plus élevées, puis coulage du mélange fondu dans un moule (coupelle ou doigt de gant). Elles peuvent aussi être obtenues par extrusion comme décrit dans la demande EP-A-0 667 146.

L'invention est illustrée plus en détail dans les exemples suivants. Les pourcentages sont des pourcentages en poids.

**Exemple : Produit de rouge à lèvres**

Première composition

| | |
|---|---|
| Cire de polyéthylène (Masse moléculaire en poids 500) | 10,5 % |
| Alcools gras linéaires (PERFORMACOL 550 ALCOHOL commercialisé par New Phases Technologies) | 2,5 % |
| Dispersion de l'exemple 1 | 68 % |
| Acetate isobutyrate de saccharose (EASTMAN SAIB commercialisé par EASTMAN CHEMICAL) | 5 % |
| Pâte pigmentaire | 13,5 % |
| Parfum | 0,5 % |

On prépare une dispersion de copolymère non réticulé d'acrylate de méthyle et d'acide acrylique dans un rapport 95/5, dans l'isododécane, selon la méthode de l'exemple 1 du document EP-A-749 746, en remplaçant l'heptane par l'isododécane. On obtient ainsi une dispersion de particules de poly(acrylate de méthyle/acide acrylique) stabilisées en surface dans l'isododécane par un copolymère dibloc séquence polystyrène/copoly(éhylène-propylène) vendu sous le nom de Kraton G1701, ayant un taux de matière sèche de 25% en poids.

Dans un poêlon on introduit la cire de polyéthylène, les alcools en C30-50 et la pâte pigmentaire que l'on chauffe à 100°C sous agitation magnétique afin d'obtenir un mélange homogène. La pâte pigmentaire est constituée de 70 % de pigments, 1 % de stéarate d'acide poly(12-hydroxystéarique) et 29 % de polyisobutène hydrogéné. On ajoute ensuite la dispersion selon l'exemple 1 et l'acétate isobutyrate de saccharose tout en maintenant la température et l'agitation jusqu'à homogénéisation du mélange. La composition est coulée dans des moules. Les sticks obtenus sont homogènes en teinte et glissant à l'application. Ils conduisent à un dépôt sur les lèvres de bonne tenue, ne migrant pas, non transfert, ne collant pas.

Deuxième composition

| | |
|---|---|
| Polydiméthylsiloxane commercialisée sous la référence AK 300000 par Wacker (300 000 cSt ) | 33 % |
| Polydiméthylsiloxane commercialisée sous la reference DC 200 (350 cSt) par Dow Corning | 43% |
| Mélange de polydiméthylsiloxane alpha-oméga dihydroxylée et de polydiméthylsiloxane 5 cSt commercialisé sous la référence DC 2-9085 par Dow Corning | 14% |
| Pentacyclosiloxane | 10% |

La tenue du produit de maquillage est évaluée selon le protocole décrit précédemment. Elle est égale à 69%.

Autre exemple de deuxième composition

| | |
|---|---|
| Polydiméthylsiloxane commercialisée sous la référence DOW CORNING 200 FLUID 60000 cSt | 50% |
| Polydiméthylsiloxane commercialisée sous la référence DOW CORNING 200 FLUID 350 CST | 43% |
| Cyclohexadiméthylsiloxane DOW CORNING 246 FLUID | 7% |

La tenue du produit de maquillage est évaluée selon le protocole décrit précédemment. Elle est égale à 73%.

## Revendications

1. Produit cosmétique, notamment de maquillage, comprenant une première composition solide et une seconde composition destinée à être appliquée sur la première, la première composition comprenant un milieu physiologiquement acceptable, et la seconde composition comprenant au moins un premier polymère siliconé de viscosité à 25°C mesurée selon la norme ASTM D-445 comprise entre 5 et 10 000 cSt, le premier polymère siliconé étant tel que lorsqu'il est en quantité suffisante dans la seconde composition, le rapport entre i) la tenue d'un dépôt dudit produit résultant de l'application successive des première et seconde compositions et ii) la tenue d'un dépôt de la première composition est supérieur à au moins 1,1, la tenue d'un dépôt de ladite première composition étant supérieure ou égale à 30%.

2. Produit cosmétique, notamment de maquillage, comprenant une première composition solide et une seconde composition destinée à être appliquée sur la première, la première composition comprenant un milieu physiologiquement acceptable, et la seconde composition comprenant au moins un premier polymère siliconé choisi parmi les polydiméthylsiloxanes de viscosité à 25°C mesurée selon la norme ASTM D-445 comprise entre 5 et 10 000 cSt et au moins un deuxième polymère siliconé choisi parmi les polydiméthylsiloxanes de viscosité à 25°C mesurée selon la norme ASTM D-445 comprise entre 5 000 et 300 000 cSt et supérieure à la viscosité dudit premier polymère.

3. Produit cosmétique, notamment de maquillage, comprenant une première composition solide et une seconde composition destinée à être appliquée sur la première, la première composition comprenant un milieu physiologiquement acceptable, et la seconde composition contenant un premier polymère siliconé de viscosité à 25°C mesurée selon la norme ASTM D-445 comprise entre 50 et 1 000 cSt et un second polymère siliconé de viscosité à 25°C mesurée selon la norme ASTM D-445 comprise entre 30 000 et 300 000 cSt.

4. Produit cosmétique, notamment de maquillage, comprenant une première composition solide et une seconde composition destinée à être appliquée sur la première, la première composition comprenant un milieu physiologiquement acceptable, et la seconde composition comprenant au moins un premier polymère siliconé de viscosité à 25°C mesurée selon la norme ASTM D-445 comprise entre 5 et 10 000 cSt, le premier polymère siliconé étant tel que lorsqu'il est en quantité suffisante dans la seconde composition, la tenue d'un dépôt dudit produit résultant de l'application successive des première et seconde compositions, est supérieure à 65%.

5. Produit cosmétique, notamment de maquillage, comprenant une première composition et une seconde composition destinée à être appliquée sur la première, la première composition comprenant un milieu physiologiquement acceptable et un polymère acrylique, et la seconde composition comprenant au moins un premier polymère siliconé de viscosité à 25°C mesurée selon la norme ASTM D-445 comprise entre 5 et 10 000 cSt, le premier polymère siliconé étant tel que lorsqu'il est en quantité suffisante dans la seconde composition, le rapport entre i) la tenue d'un dépôt dudit produit résultant de l'application successive des première et seconde compositions et ii) la tenue d'un dépôt de la première composition est supérieur à au moins 1,1, la tenue d'un dépôt de ladite première composition étant supérieure ou égale à 30%.

6. Produit cosmétique, notamment de maquillage, comprenant une première composition et une seconde composition destinée à être appliquée sur la première, la première composition comprenant un milieu physiologiquement acceptable et un polymère acrylique, et la seconde composition comprenant au moins un premier polymère siliconé choisi parmi les polydiméthylsiloxanes de viscosité à 25°C mesurée selon la norme ASTM D-445 comprise entre 5 et 10 000 cSt et au moins un deuxième polymère siliconé choisi parmi les polydiméthylsiloxanes de viscosité à 25°C mesurée selon la norme ASTM D-445 comprise entre 5 000 et 300 000 cSt et supérieure à la viscosité dudit premier

polymère.

**7.** Produit cosmétique, notamment de maquillage, comprenant une première composition et une seconde composition destinée à être appliquée sur la première, la première composition comprenant un milieu physiologiquement acceptable et un polymère acrylique, et la seconde composition contenant un premier polymère siliconé de viscosité à 25°C mesurée selon la norme ASTM D-445 comprise entre 5 et 10 000 cSt et un second polymère siliconé de viscosité à 25°C mesurée selon la norme ASTM D-445 comprise entre 30 000 et 300 000 cSt.

**8.** Produit cosmétique, notamment de maquillage, comprenant une première composition et une seconde composition destinée à être appliquée sur la première, la première composition comprenant un milieu physiologiquement acceptable et un polymère acrylique, et la seconde composition comprenant au moins un premier polymère siliconé de viscosité à 25°C mesurée selon la norme ASTM D-445 comprise entre 5 et 10 000 cSt, le premier polymère siliconé étant tel que lorsqu'il est en quantité suffisante dans la seconde composition, la tenue d'un dépôt dudit produit résultant de l'application successive des première et seconde compositions, est supérieure à 65%.

**9.** Produit selon la revendication 1 ou 5, **caractérisé en ce que** le rapport entre la tenue d'un dépôt du produit et la tenue de la première composition est supérieure ou égale à 1,15, notamment supérieure ou égale à 1,20.

**10.** Produit selon la revendication 1, 5 ou 9, **caractérisé en ce que** la tenue d'un dépôt de la première composition est supérieure ou égale à 40%, notamment supérieure ou égale à 50%, notamment supérieure ou égale à 55%.

**11.** Produit selon l'une des revendications 1, 2, 4, 5, 6, 8 ou l'une des revendications qui en dépendent, **caractérisé en ce que** le premier polymère siliconé a une viscosité comprise entre 50 et 5 000 cSt.

**12.** Produit selon la revendication précédente, **caractérisé en ce que** le premier polymère siliconé a une viscosité comprise entre 10 et 1 000 cSt, notamment entre 100 et 500 cSt.

**13.** Produit selon l'une des revendications précédentes, **caractérisé en ce que** la seconde composition a une viscosité à 25°C comprise entre 3 et 25 Pa.s.

**14.** Produit selon l'une des revendications 1, 4, 5, 8 ou l'une des revendications qui en dépendent, **caractérisé en ce que** la seconde composition contient un seul polymère siliconé et que sa viscosité à 25°C est comprise entre 3 et 6 Pa.s.

**15.** Produit selon la revendication 1, 4, 5 ou 8, **caractérisé en ce que** la deuxième composition contient un second polymère siliconé de viscosité comprise entre 5 000 et 300 000 cSt et supérieure à la viscosité du premier polymère siliconé.

**16.** Produit selon la revendication 2, 3, 6, 7, 15 ou l'une des revendications qui en dépendent, **caractérisé en ce que** le deuxième polymère siliconé a une viscosité comprise entre 10 000 et 200 000 cSt, par exemple entre 30 000 et 100 000 cSt.

**17.** Produit selon la revendication 2, 3, 6, 7, 15 ou l'une des revendications qui en dépendent, **caractérisé en ce que** le premier et le deuxième polymères siliconés sont dans une proportion telle que la viscosité dynamique de la deuxième composition à 25 °C, mesurée avec un viscosimètre rotatif Brookfield RV, est comprise entre 5 à 25 Pa.s.

**18.** Produit selon la revendication 4, 8 ou l'une des revendications qui en dépendent, **caractérisé en ce que** la tenue du produit est supérieure ou égale à 70%, notamment supérieure à 75%.

**19.** Produit selon l'une des revendications 1, 3, 4, 5, 7, 8 ou de l'une des revendications qui en dépendent, **caractérisé en ce que** le premier polymère siliconé est choisi parmi les polyalkylsiloxanes, en particulier les polydiméthylsiloxanes.

**20.** Produit selon la revendication 15 ou l'une des revendications qui en dépendent, **caractérisé en ce que** le deuxième polymère siliconé est choisi parmi les polyalkylsiloxanes, en particulier les polydiméthylsiloxanes.

**21.** Produit selon l'une des revendications précédentes, **caractérisé en ce que** la deuxième composition est transparente.

**22.** Produit selon l'une des revendications précédentes, **caractérisé en ce que** la deuxième composition contient en outre un agent de coloration, choisi de préférence parmi les nacres, les pigments à effet interférentiel et les pigments à reflet métallique.

**23.** Produit selon l'une des revendications précédentes, **caractérisé en ce que** la deuxième composition est exempte de cire.

**24.** Produit selon l'une des revendications précédentes, **caractérisé en ce que** la deuxième composition est liquide.

**25.** Produit selon l'une des revendications précédentes, **caractérisé en ce que** la deuxième composition contient moins de 10% en poids de pigments et/ou de charges.

**26.** Produit selon l'une des revendications précédentes, **caractérisé en ce que** la deuxième composition contient moins de 50% en poids d'une huile siliconée ayant une viscosité à 25°C mesurée selon la norme ASTM D-445 inférieure à 50 cSt, de préférence moins de 40%, de préférence moins de 30%, de préférence moins de 20% en poids.

**27.** Produit selon l'une des revendications 1 à 4 ou l'une des revendications qui en dépendent, **caractérisé en ce que** la première composition comprend un polymère acrylique.

**28.** Produit selon la revendication 27, **caractérisé en ce que** la première composition comprend une phase grasse liquide et un polymère acrylique sous la forme de particules dispersées dans ladite phase liquide.

**29.** Produit selon l'une des revendications 27 ou 28, **caractérisé en ce que** le polymère éthylénique représente 2 à 40% en poids du poids total de la première composition, de préférence de 5 à 35% et mieux encore de 8 à 30%.

**30.** Produit selon la revendication 28, **caractérisé en ce que** la première composition contient un stabilisant.

**31.** Produit selon la revendication précédente, **caractérisé en ce que** le stabilisant est un polymère bloc greffé ou séquencé, comprenant au moins un bloc résultant de la polymérisation de diène et au moins un bloc d'un polymère vinylique.

**32.** Produit selon la revendication précédente, **caractérisé en ce que** le stabilisant est un polymère dibloc.

**33.** Produit selon l'une des revendications 27 à 32, **caractérisée en ce que** le polymère acrylique est un polymère acrylique greffé.

**34.** Produit selon l'une des revendications précédentes, **caractérisé en ce que** la première composition est sous forme anhydre.

**35.** Produit selon l'une des revendications précédentes, **caractérisé en ce que** la deuxième composition est sous forme anhydre.

**36.** Produit selon l'une des revendications précédentes, **caractérisé en ce qu'**il se présente sous la forme d'un fond de teint, d'un fard à joues ou à paupières, d'un rouge à lèvres, d'un produit ayant des propriétés de soin, d'un eye-liner, d'un produit anti-cernes, ou d'un produit de maquillage du corps.

**37.** Produit selon la revendication précédente, **caractérisé en ce qu'**il se présente sous la forme d'un rouge à lèvres.

**38.** Produit selon l'une des revendications 5 à 8 ou l'une des revendications qui en dépendent, **caractérisé en ce que** la première composition est liquide.

**39.** Kit de maquillage comprenant un produit selon l'une des revendications 1 à 38.

**40.** Procédé de maquillage de la peau et/ou des lèvres et/ou des phanères consistant à appliquer sur la peau, les lèvres et/ou les phanères une première couche d'une première composition comprenant un milieu physiologiquement acceptable et un polymère acrylique, puis à appliquer sur tout ou partie de ladite première couche, une seconde couche d'une seconde composition comprenant au moins un polymère siliconé de viscosité à 25°C mesurée selon la norme ASTM D-445 comprise entre 5 et 10 000 cSt, et la tenue d'un dépôt résultant de l'application successive

de la première composition et de la deuxième composition étant supérieure à 65%.

**41.** Utilisation d'une deuxième composition comprenant au moins un polymère siliconé de viscosité à 25°C mesurée selon la norme ASTM D-445 comprise entre 5 et 10 000 cSt, destinée à être appliquée sur une première composition comprenant un milieu physiologiquement acceptable et un polymère acrylique, pour obtenir un produit cosmétique dont un dépôt résultant de l'application successive de la première et de la deuxième composition présente une tenue supérieure à 65%.

**42.** Procédé de maquillage de la peau et/ou des lèvres et/ou des phanères consistant à appliquer sur la peau, les lèvres et/ou les phanères une première couche d'une première composition solide comprenant un milieu physiologiquement acceptable, puis à appliquer sur tout ou partie de ladite première couche, une seconde couche d'une seconde composition comprenant au moins un polymère siliconé de viscosité à 25°C mesurée selon la norme ASTM D-445 comprise entre 5 et 10 000 cSt, et la tenue d'un dépôt résultant de l'application successive de la première composition et de la deuxième composition étant supérieure à 65%.

**43.** Utilisation d'une deuxième composition comprenant au moins un polymère siliconé de viscosité à 25°C mesurée selon la norme ASTM D-445 comprise entre 5 et 10 000 cSt, destinée à être appliquée sur une première composition solide comprenant un milieu physiologiquement acceptable, pour obtenir un produit cosmétique dont un dépôt résultant de l'application successive de la première et de la deuxième composition présente une tenue supérieure à 65%.